Europäisches Patentamt

European Patent Office

Office européen des brevets

(11) Publication number: **0 450 065 A1**

# EUROPEAN PATENT APPLICATION
## published in accordance with Art. 158(3) EPC

(21) Application number: 89900652.2

(22) Date of filing: 16.12.88

(86) International application number:
PCT/JP88/01280

(87) International publication number:
WO 90/06756 (28.06.90 90/15)

(51) Int. Cl.⁵: **A61K 33/24**

(43) Date of publication of application:
09.10.91 Bulletin 91/41

(84) Designated Contracting States:
**BE CH DE FR GB IT LI NL SE**

(71) Applicant: **TERUMO KABUSHIKI KAISHA**
**No. 44-1, Hatagaya 2-chome, Shibuya-ku**
**Tokyo 151(JP)**

Applicant: **Fujita, Haruhisa**
**11-8, Shoudo 2-chome, Sakae-ku**
**Yokohama-shi, Kanagawa-ken(JP)**

Applicant: **Yamase, Toshihiro**
**13-905, Wakabadai 4-chome, Asahi-ku**
**Yokohama-shi, Kanagawa-ken(JP)**

Applicant: **Fukushima, Kouji**
**23-5-3, Hijirigaoka 2-chome**
**Tama-shi, Tokyo(JP)**

Applicant: **Seto, Yoshiko**
**2-3-406, Natsumidai 1-chome**

**Funabashi-shi, Chiba-ken(JP)**

(72) Inventor: **FUJITA, Haruhisa 11-8, 2-chome**
**Shoudo Sakae-ku**
**Yokohama-shi Kanagawa 247(JP)**
Inventor: **YAMASE, Toshihiro 13-905, 4-chome**
**Wakabadaí Asahi-ku**
**Yokohama-shi Kanagawa 241(JP)**
Inventor: **FUKUSHIMA, Kouji 23-5-3, 2-chome**
**Hijirigaoka Tama-shi**
**Tokyo 206(JP)**
Inventor: **SETO, Yoshiko 2-3-406, 1-chome**
**Natsumidai Funabashi-shi**
**Chiba 273(JP)**
Inventor: **FUKUMA, Mariko 4-7, 5-chome**
**Sekimae Musashino-shi**
**Tokyo 180(JP)**

(74) Representative: **Gillard, Marie-Louise et al**
**Cabinet Beau de Loménie 55, Rue**
**d'Amsterdam**
**F-75008 Paris(FR)**

(54) **ANTIVIRAL AGENT.**

(57) An antiviral agent, which contains as an active ingredient a salt of heteropolyacid ion represented by the general formula: $[XM_{12}O_{40}]^{P-}$, wherein X represents an ion selected from the group consisting of groups III to VI elements of the periodic table and transition metals, M represents one, or a mixture of up to three, of molybdenum, tungsten, aluminum, vanadium, niobium, tantalum, cobalt, nickel and titanium, O represents oxygen, and p represents a positive integer.

Technical Field

This invention relates to an antiviral agent, particularly an agent to make herpesvirus ineffective, which exhibits an antiviral activity and possesses as an active component thereof a medicinally useful heteropolyoxymetalate.

Background Art

No heretofore known compound has manifested through generalized administration a clear therapeutic effect actually on a virus disease. Recently, an acyclovir (ACV) has been discovered and a anti-herpes virus agent fit for generalized administration has been developed. The bottleneck in the study on an antiviral agent resides in the fact that an effective antiviral agent of high selective toxicity is not easily obtained because a virus is a strictly intracellular parasite and the mechanism of propagation of a virus depends virtually wholly on the host cell. In the various pathogenic viruses inductive of diseases in man and beasts, the group of herpesviruses including herpes simplex virus (HSV) cause recurrent infections. The development of vaccines effective on these herpesviruses, therefore, has many problems yet to be solved. A desire has been expressed to develop a highly satisfactory antiviral agent useful for combating the herpesviruses. It has been held heretofore that not less than 90% of the cases of HSV infection excepting the cases involving infants turn out to be unapparent in type. In recent years, the necessity for establishing a method for preventing and curing a HSV infection has come to find growing recognition and excite social concern because the cases of infection through sexual intercourse of initially infected adults are increasing in consequence of the advance of age group experiencing initial infection and the cases of neonatal herpes disease due to horizontal or vertical infection are likewise increasing, because the patients observed to be in the state of immunodeficiency are increasing and are frequently exposed to the danger of developing a serious HSV infection in spite of the advance of the medical techniques, and because the relation between the cervical cancer and the HSV has come to be gradually elucidated. In the circumstances, the acyclovir (ACV) which exhibits relatively high selective toxicity and renders the general therapy of the HSV infection feasible is achieving results of therapeutic effect.

The ACV nevertheless has a problem. This problem contains the fact that the ACV, in spite of a conspicuous activity thereof manifested in resisting the HSV, incurs relatively early appearance of a resistant virus. This seems to be a phenomenon common to the ACV and other compounds similar to the component bases of nucleic acids possessing a HSV-resisting activity. In the case of the ACV, the manifestation of this activity comprises phosphorylation of the ACV into acyclo-GMP through the agency of thymidine kinase [TK(v)] capable of being induced by herpesvirus into infectious cells, transformation of the acyclo-GMP into acyclo-GTP through the agency of cellular thymidine kinase [TK(c)], conspicuous inhibition of the activity of a DNA-synthesizing enzyme by the acyclo-GTP, incorporation of the acyclo-GTP in the terminal of a DNA chain in process of synthesis and consequent formation of an end point of the DNA chain, and ensuant termination of the DNA synthesis.

Since the ACV possesses more than 200 times as high affinity for TK(v) as for TK(c), it is phosphorylated much more quickly and more copiously in HSV-infected cells than in uninfected cells. This fact accounts for the high selective toxicity or the specific anti-herpes activity and, in the meantime, induces the appearance of a resistant virus. Viruses which are abnormal to TK or a DNA polymerase exhibit resistance to the ACV. Among the fundamental and clinical findings made to date are counted reports purporting to demonstrate the presence of HSV strain lacking TK in the clinically separated virus strains. The ACV does not act upon these virus strains. The following reports have been published to date concerning the antiviral activity of polyoxymetalate ion compounds.

(1) Polyoxotungstate manifests an antiviral effect in vitro on a Rabies virus. Since this compound inhibits a reverse transcription enzymatic activity originating in the mouse mammary tumor virus, it is expected to be effective on human retrovirus. In fact, the effectiveness of this compound as HPA-23 on the AIDS virus has been reported.

(2) The effectiveness of ammonium-5-tungsto-2,0,2-antimonate on mice infected with encephalomyocartitis virus or testicular stomatitis virus has been reported.

(3) Heteropolyanion-5-tungsto-2-antimonate and 21-tungsto-9-antimonate have been reported to be effective on urine leukemia virus or murine sarcoma virus. They manifest an antiviral activity as an inhibitor of the reverse transcription enzymatic activity of mouse retrovirus.

The medicine (ACV) which is effective against the anti-herpes infectious disease as described above has been developed and put to practical use. Since this anti-herpes agent has relatively low toxicity (adverse side effect), it may well be said that the medicine has realized in a measure the therapy of the acute infectious disease of herpesvirus. The ACV, however, has such unsolved problems as the aforementioned appearance of a resistant virus and the lack of effectiveness against the TK virus. In the light of the specific mode of infection of this virus, i.e. recurrence of disease caused by reactivation of the latent infection virus, it is logical to conclude that the final objective of the control of the infectious disease caused by the herpesvirus resides in preventing the infection itself and extirpating the virus. Since early development of a vaccine fulfilling the objective is not easily attained in the existing circumstances, a desire is expressed in the industry to develop an antiviral agent possessing a new type of anti-herpesvirus activity involving a different mechanism of activity and a different site of activity.

An object of this invention, therefore, is to provide a novel antiviral agent possessing a nature fulfilling the aforementioned objective and differing entirely from the compounds associated with the component bases of nucleic acids. Another object of this invention is to provide an antiviral agent possessing a strong antiviral activity and producing very weak adverse side effects.

Disclosure of Invention

These objects are accomplished by an antiviral agent having as an active component thereof a salt of a heteropoly-anion represented by the following general formula I:

$$[XM_{12}O_{40}]^{p-} \quad \text{(I)}$$

wherein X is an ion of at least one element selected from the group consisting of the transition metals of Groups III to VI in the Periodic Table of Elements, M is one member or a mixture of up to three members selected from the group consisting of the ions severally of molybdenum, tungsten, aluminum, vanadium, niobium, tantalum, cobalt, nickel, and titanium, O is oxygen atom, and P is a positive integer.

The antiviral agent of this invention prepared in the form of an aqueous solution is sufficiently stable chemically when the pH value thereof is in the range of from 4 to 8.

The sufficient chemical stability indicates that the time-course change of the aqueous solution of the polyoxometalate at room temperature is virtually nil because this change is determined by measuring the electric current which reflects an electrochemical reduction of the polyoxometalate ion. To be specific, this determination is carried out by preparing aqueous solutions of 0.1 M polyoxometalate adjusted pH 4 to 8 (with $HClO_4$, HCl, and other buffer solutions) and subjecting these aqueous solutions to derivative pulse polarography with a dropping-mercury electrode (using a EG&G PRA-174A + 303 polarographic meter). A sample in which the peak value of electric current measured as described above at 2.5° C is retained above 70% over a period of one hour following the start of the test is reported as possessing sufficient chemical stability.

Best Mode for Carrying Out the Invention

Pentapotassium dodecatungsto borate and heptapotassium dititanate decatungsto phosphate which are used in the antiviral agent (particularly the agent effective against herpesvirus) of this invention are salts of Keggin type heteropolyanions represented by the general formula, $[XM_{12}O_{40}]^{p-}$ wherein X is one member or a mixture of up to three members selected from the group consisting of the ions severally of molybdenum, tungsten, aluminum, vanadium, niobium, tantalum, cobalt, nickel, and titanium, O is oxygen atom, and P is positive integer).

The polyoxotungstates to be used in this invention are oxo acid ion polycyclic complexes of a structure such that heteropolyoxometalates of a kind which are cluster compounds are joined through the medium of edges or apexes as basic unit structures generally having four or six oxygen atoms attached to a tungsten atom. The polyoxometalate ions in these heteropolyoxometalate have the following general characteristic features.

(1) They have an ion size in the range of from 6 to 25 Å and a molecular weight in the range of from 1,000 to 10,000.
(2) Most of them are obtained in the form of crystals exhibiting high solubility in water and polar solvents.
(3) They have a large number of hydrate molecules.
(4) They are strong oxidizing agents (serving as a polyelectron pooling agent).

The polyoxometalate ions have the nature of effecting a photoredox reaction in a solid or a solution and, therefore, are confidently expected to find commercial utility in applications as to display function devices. Owing to their high reactivity, these compounds are estimated to possess a physiological activity. As the result of various studies, it has been found that polyoxometalate ions such as, for example, polyoxotungstates, possess a highly conspicuous antiviral effect (particularly against herpesvirus). They are expected to manifest effectiveness especially against various diseases caused by human retrovirus represented by the acquired immunological deficiency syndrome (AIDS) virus which has been raising a serious social issue. This expectation logically issues from the fact that the polyoxotungstates possess an activity of inhibiting the reverse transcriptase of the virus.

The polyoxotungstates answering the description include the salts of cations severally of sodium, potassium, cesium, calcium, strontium, barium, ammonium, hydrogen, methyl ammonium, ethyl ammonium, dimethyl ammonium, trimethyl ammonium, tetramethyl ammonium, diethyl ammonium, isopropyl ammonium, diisopropyl ammonium, propyl ammonium, dipropyl ammonium, butyl ammonium, dibutyl ammonium, tributyl ammonium, tetrabutyl ammonium, and guanidinium, for example.

The heteropolyoxometalate salts of this invention include the following compounds, as follows:

1. $[XW_{12}O_{40}]^{p-}$ : X = H, $H_2$, B, Al, Ga(III), P(V), As(V), Cr(III), Mn(IV), Fe(III), Co(III), Co(II), Cu(II), Cu(I), Zn, Se(IV), Te(IV), Sb(III), Bi(III).

2. $[XW_{12}O_{40}]^{p-}$ : X = Si, Ge(IV), P(V), As(V), V(V), Ti(IV), Zn(IV), In(III), $H_2$, Mo.

3. $[PVMo_{12-x}O_{40}]^{(3+x)-}$ : x = 1~3;
$[PVW_{12-x}O_{40}]^{(3+x)-}$ : x = 1~4,

4. $[ZnW_{11}CoO_{40}]^{9-}$,
$[ZnW_{11}CoO_{40}]^{10-}$,
$[GaW_{11}CuO_{40}]^{10-}$,
$[GaW_{11}GaO_{40}]^{8-}$,
$[PW_{10}Ti_2O_{40}]^{7-}$,
$[PW_{11}TiO_{40}]^{6-}$,

5. $[(n-C_4H_9)_4N]_4 SiMo_{12}O_{40}$,
$H_4GeMo_{12}O_{40} \cdot Na_4GeMo_{12}O_{40}$,
$[(n-C_4H_9)_4N]_4 GeMo_{12}O_{40}$,
$Na_2HPMo_{12}O_{40}$,
$[(n-C_4H_9)_4N]_4 PMo_{12}O_{40}$,
$[(n-C_4H_9)_4N]_4 AsMo_{12}O_{40}$,
$K_5BW_{12}O_{40}$,
$[(n-C_4H_9)_4N]_4 HBW_{12}O_{40}$,
$H_4SiW_{12}O_{40} \cdot K_4SiW_{12}O_{40}$,
$Ba_2SiW_{12}O_{40}$,
$[(CH_3)_4N]_4 SiW_{12}O_{40}$,
$[(n-C_4H_9)_4N]_4 SiW_{12}O_{40}$,
$(NH_4)SiW_{12}O_{40}$,
$H_4GeW_{12}O_{40} \cdot K_4GeW_{12}O_{40}$,
$[(n-C_4H_9)_4N]_4 GeW_{12}O_{40}$,
$H_3PW_{12}O_{40} \cdot Na_3PW_{12}O_{40}$,
$Na_3AsW_{12}O_{40}$
$[(n-C_4H_9)_4N]_4 AsW_{12}O_{40}$
$K_6[SiNiW_{11}O_{40}H_2]$

The antiviral agent (particularly against herpesvirus) of this invention generally contains in a solid carrier or a liquid carrier, preferably a liquid carrier, at least one of the aforementioned compounds of the general formula $[XM_{12}O_{40}]^{p-}$. The compounds conforming to this invention can be used in combination with known active substances. The antiviral agent of this invention may be administered in any suitable and/or conventional mode. When such a compound of this invention is administered through a nonoral, hypodermic, intravenous, or intra-abdominal path, the carrier for the compound is an aseptic liquid such as distilled water or physiological saline solution, particularly water. Generally, the water carrier of the injection grade aqueous solution of the compound contains the active substance in a concentration of 10 mg/ml (1.0%). The compound of this invention can be administered orally. It can be used advantageously in the form of ointment or eye lotion. The oral administration may be effected more suitably in the form of tablets, capsules, powder, or suspension, for example. In these modes of administration, the active component is desired to account for at least a concentration in the range of from 1.0 to 3.0% (by weight) based on the amount of the composition. The daily dose of the compound is generally in the range of from 100 mg to 3,000 mg per adult.

Now, the present invention will be described more specifically below with reference to working examples.

4

Synthesis 1

In a glass beaker, 100 g of sodium tungstate and 5 g of boric acid were dissolved in 100 ml of purified water and the resultant solution was adjusted to pH 2.0 by addition of 6N-hydrochloric acid solution. The acid solution was refluxed for about five hours and cooled. The filtrate was again adjusted with hydrochloric acid to pH 2.0, then refluxed for 30 minutes, mixed with 20 g of potassium chloride, and left cooling. The precipitate consequently formed was separated by filtration, washed twice with diethyl ether, and recrystallized twice from warm water. The crystals consequently obtained were found by elementary analysis, thermal analysis, and NMR spectrometry to be $K_5BW_{12}O_{40} \cdot 15H_2O$ (compound A).

Synthesis 2

In a glass flask, 6 g of sodium dihydrogen sulfate and 30 g of sodium tungstate were dissolved in 100 ml of purified water. The resultant solution was kept stirred and 1.8 ml of titanium tetrachloride was slowly added dropwise thereto. The solution was refluxed for 20 minutes and immediately filtered. The filtrate was mixed with 30 g of potassium chloride. The precipitate consequently formed was separated by filtration and recrystallized twice from warm water. By elementary analysis, thermal analysis, and NMR spectrometry, the crystals were found to be $K_7PW_{10}Ti_2O_{40} \cdot 6H_2O$ (compound B).

Example 1

In vitro cellular toxicity of potassium polyoxotungstate on vero cells

Compound A and Compound B were severally dissolved in distilled water in a concentration of 5 mg/ml, to prepare master solutions. Each of the master solutions was suitably diluted with an Eagle's MEM culture medium containing 10% fetal bovine serum. A 0.2-ml portion of the resultant dilution product was placed on vero cells cultured in advance on a microplate and grown in a monolayer, cultured further thereon at 37°C for three to five days. Samples taken severally after three days'and five days'culture were placed under a microscope and tested for cytopathogenic effect brought about by a chemical agent.

The degree of cytopathogen was rated on the four-point scale, wherein + + + is virtually perfect extirpation of cells, + + is strong cytopathogen observed in spite of persistence of cellular form, + is cytopathogen observed slightly and yet clearly as compared with a control (untreated sample), and - is virtually no change observed in cellular form.

The results of the test are shown in Table 1.

Table 1

| Compound | Cellular toxity (3 days) µg/ml | | | | | | Maximum nontoxic does µg/ml | Cellular toxity (5 days) µg/ml | | | | | | Maximum nontoxic does µg/ml |
|---|---|---|---|---|---|---|---|---|---|---|---|---|---|---|
| | 5 | 10 | 20 | 50 | 100 | 200 | | 5 | 10 | 20 | 50 | 100 | 200 | |
| Compound A | - | - | - | - | - | - | >200 | - | - | - | - | - | - | >200 |
| Compound B | - | - | - | - | - | - | >200 | - | - | - | - | - | - | >200 |

It is clearly noted from the test results of Table 1 that the maximum nontoxic doses (MNTD) of Compound A and Compound B were both above the concentration of 200 μg/ml.

Example 2

Activity of potassium polyoxotungstate on propagation of vero cells

In culture tubes, vero cells were placed in varying ratios falling in the range of (7.8 to 0.7) x 10.4/0.9 ml and separately prepared solutions of Compound A and Compound B (prepared with an Eagle's MEM culture medium containing 10% fetal bovine serum) were added each in a volume of 0.1 ml to produce samples having final concentrations of 100, 200, and 500 $\mu$g/ml and cell numbers of (7.8 to 0.7) x $10^4$/ml. Samples of the control group contained 0.1 ml of a culture solution (an Eagle's MEM culture medium containing 10% fetal bovine serum).

The test was conducted by using three culture tubes per group, culturing the cells in the tubes at 37°C for three days, and taking count of live cells by the dye-exclusion method.

The results of this test are shown in Table 2.

## Table 2

| Test group | | Concentration μg/ml | Number of cells (/ tube) (x10$^4$) | | | | Average ± SDn-1 (x10$^4$) | Ratio (%) |
|---|---|---|---|---|---|---|---|---|
| Control | 0hr | | 7.1, | 8.4, | 8.0 | | 7.8 ± 0.7 | |
| | 72hr | | 82.8, | 87.6, | 85.0, | 89.5 | 86.2 ± 2.9 | |
| Compound A | | 100 | 86.4, | 88.4, | 93.7 | | 89.5 ± 3.8 | 0 |
| | | 200 | 92.8, | 83.2, | 87.0 | | 87.7 ± 4.8 | 0 |
| | | 500 | 57.7, | 51.9, | 55.5 | | 55.0 ± 2.9 | 36.2 |
| Compound B | | 100 | 90.1, | 88.1, | 85.7 | | 88.0 ± 2.2 | 0 |
| | | 200 | 95.0, | 92.8, | 98.3 | | 95.4 ± 2.8 | 0 |
| | | 500 | 69.7, | 79.7, | 74.5 | | 74.6 ± 5.0 | 13.5 |

SDn-1 : Average standard deviation

Compound A and Compound B at concentrations of 100 and 200 μg/ml exerted absolutely no effect on propagation of vero cells. At a concentration of 500 μg/ml, Compound A and Compound B showed control ratios respectively of 36.2% and 13.5% on the propagation of vero cells.

These results agree satisfactorily with the results of MNTD (maximum nontoxic dose) exceeding 200 μg/ml obtained in Example 1.

Example 3

In vitro antiviral activity of Compound A against herpes simplex virus type 1 (HF strain)
Compound A was tested for antiviral effect by the following two methods.

(1) Control of CPE (cytopathic effect) manifestation

Vero cells cultured and grown in a monolayer on microplates to test for cytopathic effect due to viral infection, with solutions of Compound A diluted to varying ratios and solutions of virus diluted to varying ratios added severally thereto in a volume of 0.1 ml, were cultured under the conditions of 5% $CO_2$ and 37°C and were examined for CPE manifestation under a microscope. The observation was continued for five to six days.

The degree of CPE brought about by viral infection was rated on the five-point scale, wherein 3 stands for CPE observed on not less than 75% of cultured cells, 2 for CPE observed on 50% of cultured cells, 1 for CPE observed on 25% of cultured cells, 0.5 for occurrence of one to two extremely minute DPE spots, and 0 for perfect absence of recognizable CPE. The CPE scores obtained at the four holes in each microplate were averaged and the difference of the average from the average obtained in the control (omitting the treatment with a chemical) was reported as ΔCPE.

(2) 50% Tissue culture infective dose ($TCID_{50}$)

The amount of virus infecting 50% of tissues ($TCID_{50}$) was determined by the Read-Munch method and expressed in negative logarithm of the maximum radio of dilution of virus. The difference of the amount thus determined from the amount found in the control (omitting the treatment with a chemical) was reported as $\Delta TCID_{50}$. A sample whose ΔCPE's found on the last day of observation and on each of the days spent for the observation exceeded 1.0 was evaluated as effective.

The results of the test are shown in Table 3.

Table 3

| Test group | Concentration μg/ml | Cytopathic effect [CPE] (3days) | | | | | Cytopathic effect [CPE] (5days) | | | | |
|---|---|---|---|---|---|---|---|---|---|---|---|
| | | CPE Score | Average | $\Delta$CPE | $TCID_{50}$ | $\Delta TCID_{50}$ | CPE Score | Average | $\Delta$CPE | $TCID_{50}$ | $\Delta TCID_{50}$ |
| HSV-1 (HF) Control | 100 | 2, 1, 2, 1 | 1.5 | . | 6.0 | 2.5 | 1, 2, 2, 2 | 1.75 | 1.75 | 6.0 | 2.5 |
| ACV | 100 | 0, 0, 0, 0 | 0 | 1.5 | 3.5 | 0.5 | 0, 0, 0, 0 | 0 | 0 | 3.5 | 0.3 |
| Compound A | 5 | 2, 1, 1, 1 | 1.25 | 0.25 | 5.5 | 0.3 | 2, 2, 1 | 1.75 | 0.5 | 5.7 | 0.3 |
| | 10 | 1, 2, 1, 1 | 1.25 | 0.25 | 5.7 | 0.3 | 1, 2, 2 | 1.25 | 0.25 | 5.7 | 0.3 |
| | 20 | 1, 1, 2, 0 | 1.25 | 0.25 | 5.7 | 0.3 | 2, 2, 0 | 1.5 | 0.25 | 5.7 | 0.3 |
| | 50 | 2, 0, 2, 1 | 1.5 | 0 | 5.7 | 0.3 | 2, 3, 0, 1 | 2.0 | 0.75 | 5.7 | 0.3 |
| | 100 | 1, 0, 1, 1 | 0.75 | 0.375 | 5.3 | 0.7 | 2, 0, 1, 1 | 1.0 | | 5.3 | 0.3 |

The antiviral effects, $\Delta TCID_{50}$, of the samples containing Compound A at varying concentrations of from 5 to 100 μg/ml, found on the fifth day of observation were invariably 0.3. In the case of ACV, a known antiviral agent, the antiviral effect, $\Delta TCID_{50}$, of the sample containing ACV in a concentration of 100 μg/ml (1/10 MNTD) was 2.5. Compound A showed a relatively weak antiviral effect on the herpes simplex virus type 1 (HF strain).

Example 4

In vitro antiviral activity of Compound A against herpes simplex virus type 2 (169 strain)

The test was carried out by following the procedure of Example 3. The results of this test are shown in Table 4.

Table 4

| Test group | Concent-ration μg/ml | Cytopathic effect [CPE] (3days) | | | | | Cytopathic effect [CPE] (5days) | | | | |
|---|---|---|---|---|---|---|---|---|---|---|---|
| | | CPE Score | Average | ΔCPE | $TCID_{50}$ | $\Delta TCID_{50}$ | CPE Score | Average | ΔCPE | $TCID_{50}$ | $\Delta TCID_{50}$ |
| HSV-2 (169) Control | | 2, 1, 2, 1 | 1.5 | | 6.5 | | 3, 3, 3, 3 | 3.0 | | 6.5 | |
| ACV | 100 | 0, 0, 0, 0 | 0 | 1.5 | 3.5 | 3.0 | 0, 0, 0, 0 | 0 | 3.05 | 3.5 | 3.0 |
| Compound A | 5 | 1, 1, 1, 1 | 1.0 | 0.5 | 6.5 | 0 | 3, 3, 2, 2 | 2.5 | 0.5 | 6.7 | 0.2 |
| | 10 | 1, 1, 1, 1 | 1.0 | 0.5 | 6.3 | 0.2 | 2, 2, 2, 2 | 2.0 | 1.0 | 6.3 | 0.2 |
| | 20 | 1, 1, 1, 1 | 1.0 | 0.5 | 6.5 | 0 | 1, 2, 1, 1 | 1.25 | 1.75 | 6.2 | 0.3 |
| | 50 | 0.5,0.5,0.5,0.5 | 0.5 | 1.0 | 5.5 | 1.0 | 0.5,0.5,0.5,0.5 | 0.5 | 2.5 | 5.5 | 1.0 |
| | 100 | 0, 0, 0, 0 | 0 | 1.5 | 3.5 | 3.0 | 0, 0, 0, 0 | 0 | 3.05 | 3.5 | 3.0 |

The antiviral effect, TCID 50, of Compount A against the herpes simplex virus type 2 (169 strain) found on the fifth day of observation was 0.2 at a concentration of 10 μg/ml, 0.3 at e concentration of 20 μg/ml, 1.0 at A concentration of 50 μg/ml, and 3.0 at A concentration of 100 μg/ml.

In contrast, the antiviral effect, ΔTCID 50, of the known antiviral agent ACV was 3.0 at a concentration of 100 μg/ml, a value equaling that of Compound A at a concentration of 100 μg/ml.

Thus, Compound A showed the same degree of antiviral effect against the herpes simplex virus type 2 (169 strain) as ACV.

Example 5

In vitro antiviral effect of Compound A against herpes simplex virus type 1 (HF strain)

The test was carried out by following the procedure of Example 3. The results of this test are shown in Table 5.

Table 5

| Test group | Concentration μg/ml | Cytopathic effect [CPE] (3 days) | | | | | Cytopathic effect [CPE] (6 days) | | | | |
|---|---|---|---|---|---|---|---|---|---|---|---|
| | | CPE Score | Average | ΔCPE | $TCID_{50}$ | $\Delta TCID_{50}$ | CPE Score | Average | ΔCPE | $TCID_{50}$ | $\Delta TCID_{50}$ |
| HSV-1 Control | | 2, 3, 3, 3 | 2.75 | | 6.3 | | 3, 3, 3, 3 | 3.0 | | 6.3 | |
| Ara A | 20 | 2, 2, 3, 3 | 2.5 | 0.25 | 6.5 | -0.2 | 3, 3, 3, 3 | 3.0 | 0 | 6.5 | -0.2 |
| IDU | 20 | 2, 2, 2, 3 | 2.25 | 0.5 | 6.3 | 0 | 3, 3, 3, 3 | 3.0 | 0 | 6.5 | -0.2 |
| ACV | 100 | 0.5, 1, 1, 1 | 0.875 | 1.875 | 5.5 | 0.8 | 1, 1, 1, 1 | 1.0 | 2.0 | 4.3 | 2.0 |
| Compound A | 100 | 0.5, 0, 0, 0, | 0.125 | 2.625 | 4.7 | 1.6 | 0, 0, 1, 1 | 0.5 | 2.5 | 5.0 | 1.3 |
| | 200 | 0, 0, 0, 0, | 0 | 2.75 | <3.5 | >2.8 | 0, 0, 0, 0 | 0 | 3.0 | <3.5 | >2.5 |

The antiviral effect, $\Delta TCID_{50}$, of Compound A on the sixth day of observation was 1.3 at a concentration of 100 μg/ml and > 2.8 at a concentration of 200 μg/ml. The antiviral effect, $\Delta TCID_{50}$, of the known antiviral agent ACV was 2.0 at a concentration of 100 μg/ml. In contrast, neither adenine arabinoside (Ara-A) nor (IDU) showed no discernible antiviral effect at a concentration of 20 μg/ml.

Thus, Compound A showed a strong antiviral activity against the herpesvirus 1 type (HF strain) and

13

effected higher control than ACV.

Example 6

In vitro antiviral activity of Compound A against herpes simplex virus type 2 (169 strain)
The test was carried out by following the procedure of Example 3. The results of this test are shown in Table 6.

Table 6

| Test group | Concentration μg/ml | Cytopathic effect [CPE] (3days) | | | | Cytopathic effect [CPE] (6days) | | | |
|---|---|---|---|---|---|---|---|---|---|
| | | CPE Score | Average ΔCPE | $TCID_{50}$ $\Delta TCID_{50}$ | | CPE Score | Average ΔCPE | $TCID_{50}$ $\Delta TCID_{50}$ | |
| HSV-2 (169) Control | | 2, 2, 3, 3 | 2.5 | 6.7 | | 3, 3, 3, 3 | 3.0 | 6.7 | |
| Ara A | 20 | 1, 1, 2, 2 | 0.5 — 1.0 | 7.0 — -0.3 | | 3, 3, 3, 3 | 3.0 — 0 | 7.5 — -0.8 | |
| IDU | 20 | 0, 1, 1, 1 | 0.75 — 1.75 | 5.3 — 1.4 | | 0, 1, 1, 3 | 1.25 — 1.75 | 5.5 — -1.2 | |
| ACV | 100 | 0, 0, 0, 0 | 0 — >2.5 | <3.5 — >3.2 | | 0, 0, 0 | 0 — 3.0 | 3.5 — 3.2 | |
| Compound A | 100 | 0, 0, 0, 0 | 0 — 2.5 | <3.5 — >3.2 | | 0, 0, 0, | 0 — 3.0 | <3.5 — >3.2 | |
| Compound A | 200 | 0, 0, 0, 0 | 0 — 2.5 | <3.5 — >3.2 | | 0, 0, 0, | 0 — 3.0 | <3.5 — >3.2 | |

The antiviral effect, $\Delta TCID_{50}$, of Compound A on the sixth day of observation was >3.2 at a concentration of 100 μg/ml and >3.2 at a concentration of 200 μg/ml, while that of the known antiviral agent ACV was 3.2 at a concentration of 100 μg/ml, that of IDU was 1.2 at a concentration of 20 μg/ml, and that of Ara-A was nil (indiscernible) at a concentration of 20 μg/ml.

Thus, Compound A showed a stronger antiviral effect against the herpes simplex virus type 2 (169 strain) than ACV.

15

Example 7

Antiviral activity of Compound A and Compound B against herpes simplex virus type 1 (KOS strain)
The test was carried out by following the procedure of Example 3. The results of this test are shown in Table 7.

Table 7

| Test group | Concentration μg/ml | Cytopathic effect [CPE] (3days) | | | | | Cytopathic effect [CPE] (5days) | | | | |
|---|---|---|---|---|---|---|---|---|---|---|---|
| | | CPE Score | Average | △CPE | $TCID_{50}$ | $\triangle TCID_{50}$ | CPE Score | Average | △CPE | $TCID_{50}$ | $\triangle TCID_{50}$ |
| HSV-1 (KOS) Control | | 2, 2, 2, 2 | 2.0 | | 5.5 | | 3, 3, 3, 3 | 3.0 | | 6.0 | |
| Ara A | 30 | 1, 1, 1, 1 | 1.0 | 1.0 | 5.0 | 0.5 | 2, 2, 2, 2 | 2.0 | 1.0 | 5.7 | 0.3 |
| IDU | 30 | 0, 1, 1, 1 | 1.0 | 1.0 | 5.3 | 0.2 | 2, 2, 2, 2 | 2.0 | 1.0 | 6.0 | 0.3 |
| ACV | 100 | 0, 0, 0, 0 | 0 | 2.0 | 3.5 | 2.0 | 0, 0, 0, 0 | 0 | 3.0 | 3.5 | 2.5 |
| Compound A | 100 | 0, 0, 0, 0 | 0 | 2.0 | 3.5 | 2.0 | 0, 0, 0, 0 | 0 | 3.0 | 3.5 | 2.5 |
| | 200 | 0, 0, 0, 0 | 0 | 2.0 | 3.5 | 2.0 | 0, 0, 0, 0 | 0 | 3.0 | 3.5 | 2.5 |
| Compound B | 100 | 0, 0, 0, 0 | 0 | 2.0 | 3.5 | 2.0 | 0, 0, 0, 0 | 0 | 3.0 | 3.5 | 2.5 |
| | 200 | 0, 0, 0, 0 | 0 | 2.0 | 3.5 | 2.0 | 0, 0, 0, 0 | 0 | 3.0 | 3.5 | 2.5 |

The antiviral effect, $\Delta TCID_{50}$, of Compound A on the fifth day of observation was 2.5 at a concentration of 100 μg/ml and 2.5 at a concentration of 200 μg/ml and that of Compound B was 2.5 at a concentration of 100 μg/ml and 2.5 at a concentration of 200 μg/ml.

In contrast, the antiviral effect of the known antiviral agent ACV was 2.5 at a concentration of 100 μg/ml and that of IDU and that of Ara-A were invariably 0.3 at a concentration of 30 μg/ml.

Thus, Compound A and Compound B showed a strong antiviral activity against the herpes simplex virus type 1 (KOS strain). Their antiviral effects were equal to the antiviral activity of the known antiviral agent ACV.

Example 8

Antiviral activity of Compound A and Compound B against one-step growth of herpes simplex virus type 1 (KOS strain)

Cells grown in a monolayer, with a sample of a given compound diluted in a varying ratio added, were pretreated for 15 minutes. Then, the pretreated cells were infected with the herpes simplex virus type 1 (KOS strain) at an infecting ratio of 0.25 and left standing for 24 hours. At the end of the standing, the intracellular and extracellular amounts of virus were determined by the plaque assay with vero cells. The results of this test are shown in Table 8.

Table 8

| Test group | Concentration μg/ml | Intracellular virus | | | | Intracellular virus | | | |
|---|---|---|---|---|---|---|---|---|---|
| | | Number of plagues /dish | Average | Control ratio % | Control ratio -logT/C | Number of plagues /dish | Average | Control ratio % | Control ratio -logT/C |
| Control | | $4.30 \times 10^8$<br>$3.75 \times 10^8$<br>$4.15 \times 10^8$ | $4.07 \times 10^8$ | | | $3.60 \times 10^7$<br>$2.40 \times 10^7$<br>$3.00 \times 10^7$ | $3.00 \times 10^7$ | | |
| Ara A | 30 | $8.75 \times 10^4$<br>$6.00 \times 10^4$ | $7.38 \times 10^4$ | >99.9 | 4.742 | $4.60 \times 10^3$<br>$3.25 \times 10^3$ | $3.93 \times 10^3$ | >99.9 | 3.883 |
| IDU | 30 | $1.30 \times 10^6$<br>$7.0 \times 10^5$ | $1.00 \times 10^6$ | >99.8 | 2.610 | $1.00 \times 10^3$<br>$5.00 \times 10^2$ | $7.50 \times 10^3$ | >99.9 | 3.602 |
| ACV | 100 | $1.05 \times 10^3$<br>$6.60 \times 10^2$ | $8.50 \times 10^2$ | >99.9 | 5.680 | $<5.00 \times 10$<br>$<5.00 \times 10$ | $<5.00 \times 10$ | >99.9 | 5.778 |
| Compound A | 50 | $1.53 \times 10^6$<br>$1.15 \times 10^6$ | $1.34 \times 10^6$ | 99.7 | 2.482 | $5.60 \times 10^5$<br>$8.65 \times 10^5$ | $7.13 \times 10^5$ | 97.6 | 1.623 |
| | 100 | $2.10 \times 10^6$<br>$1.88 \times 10^6$ | $1.99 \times 10^6$ | >99.9 | 7.211 | $7.95 \times 10^4$<br>$8.80 \times 10^4$ | $8.38 \times 10^4$ | 99.7 | 2.554 |
| | 200 | $<2.5 \times 10$<br>$<2.5 \times 10$ | $<2.5 \times 10$ | >99.9> | 7.211 | $<5.00 \times 10$<br>$<5.00 \times 10$ | $<5.00 \times 10$ | >99.9 | 5.778 |
| Compound B | 50 | $7.0 \times 10^5$<br>$5.00 \times 10^5$ | $6.00 \times 10^5$ | 99.8 | 2.831 | $5.75 \times 10^5$<br>$5.90 \times 10^5$ | $5.83 \times 10^5$ | 98.1 | 1.711 |
| | 100 | $1.38 \times 10^5$<br>$8.50 \times 10^4$ | $1.12 \times 10^5$ | >99.9 | 3.560 | $1.85 \times 10^5$<br>$2.35 \times 10^5$ | $2.10 \times 10^5$ | 99.3 | 2.155 |
| | 200 | $1.28 \times 10^3$<br>$8.50 \times 10^2$ | $1.07 \times 10^3$ | >99.9 | 5.580 | $4.60 \times 10^4$<br>$5.30 \times 10^4$ | $4.95 \times 10^4$ | 99.8 | 2.783 |

In the one-step growth of herpesvirus 1 type (KOS strain), Compound A and Compound B showed conspicuous control of both intracellular and extracellular viruses.

As regards the amount of intracellular virus, the control ratio of Compound A was 99.7% at a concentration of 50μg/ml, >99.9% at a concentration of 100 μg/ml, and >99.9% at a concentration of 200 μg/ml. The control ratio of Compound B was 99.8% at a concentration of 50 μg/ml, >99.9% at a

18

**EP 0 450 065 A1**

concentration of 100 $\mu$g/ml, and >99.9% at a concentration of 200 $\mu$g/ml. In contrast, the control ratio of Ara-A was >99.9% at a concentration of 30 $\mu$g/ml, that of IDU 99.8% at a concentration of 30 $\mu$g/ml, and that of ACV >99.9% at a concentration of 100 $\mu$g/ml.

Then, as regards the amount of extracellular virus, the control ratio of Compound A was 97.6% at a concentration of 50 $\mu$g/ml, 99.7% at a concentration of 100 $\mu$g/ml, and >99.9% at a concentration of 200 $\mu$g/ml, while that of Compound was 98.1% at a concentration of 50 $\mu$g/ml 99.3% at a concentration of 100 $\mu$g/ml, and 99.8% at a concentration of 200 $\mu$g/ml. In contrast, the control ratio of Ara-A was >99.9% at a concentration of 30 $\mu$g/ml, that of IDU >99.9% at a concentration of 30 $\mu$g/ml, and that of ACV >99.9% at a concentration of 100 $\mu$g/ml.

Thus, Compound A and Compound B both showed a strong antiviral effect aggainst the one-step growth of the herpes simplex virus type 1 (KOS strain).

Acute toxicity

In the heteropolyoxometalate ion salts represented by the general formula $[XM_{12}O_{40}]^{p-}$ Compound A and Compound B exhibited strong activity to make herpesvirus ineffective. When these compounds were severally administered intraabdominally ICR/CD-I to female mice at a dose of 2,000 mg/kg, they were not observed to manifest toxicity as evinced by decrease of body weight, for example.

Efficacy:

The salts of heteropolyoxometalate ions of this invention manifest strong antiviral effect against herpes simplex virus type 1 (HF and KOS strains) and herpes simplex virus type 2 (169 strain). This effect is equal to or higher than the effect produced against herpesvirus by ACV, the most effective antiviral agent existing today.

Compound A and Compound B control not less than 99.9% of the intracellular and extracellular viruses in the one-step growth of vero cells.

Industrial Applicability

As described above, this invention concerns an antiviral agent having as an active component thereof the salt of a heteropolyoxometalate ion represented by the general formula $[XM_{12}O_{40}]^{p-}$. As demonstrated by numerous examples, the compounds of the formula exhibit a strong antiviral activity against herpes simplex virus type 1 and herpes simplex type 2. Their antiviral effects are superior to those produced by ADV, Ara-A, IDU, etc. which have been already in clinical use.

In terms of toxicity, the compounds show very low in vitro cyto-toxicity and exhibit very low toxicity against mice. Moreover, since these compounds are readily soluble in water, they allow very easy handling and permit stable formulation of medicine.

The salts of these heteropolyoxometalate ions, therefore, are highly useful as antiviral compounds (particularly against herpes simplex viruses).

**Claims**

1. An antiviral agent having as an active component thereof a salt of a heteropolyanion represented by the general formula I:

$$[XM_{12}O_{40}]^{p-} \quad (I)$$

wherein X is an ion of at least one member selected from the group consisting of transition metals of Groups III to VI in the Periodic Table of Elements, M is one member or a mixture of up to three members selected from the group consisting of the ions severally of molybdenum, tungsten, aluminum, vanadium, niobium, tantalum, cobalt, nickel, and titanium, O is oxygen atom, and p is a positive integer.

2. An antiviral agent according to claim 1, wherein the cation of said salt of heteropolyanion is selected from the group consisting of cations severally of sodium, potassium, cesium, calcium, strontium, barium, ammonium, hydrogen, methyl ammonium, ethyl ammonium, dimethyl ammonium, trimethyl ammonium, tetramethyl ammonium, diethyl ammonium, isopropyl ammonium, diosopropyl ammonium, propyl ammonium, dipropyl ammonium, butyl ammonium dibutyl ammonium, tributyl ammonium,

19

tetrabutyl ammonium, and guanidinium.

3. An antiviral agent according to claim 1 or claim 2, wherein said virus is herpesvirus.

4. An antiviral agent according to any of claima 1 to 3, wherein said salt of heteropolyanion is in the form of an aqueous solution and said aqueous solution is stable at a pH value in the range of from 4 to 8.

# INTERNATIONAL SEARCH REPORT

International Application No PCT/JP88/01280

**I. CLASSIFICATION OF SUBJECT MATTER** (if several classification symbols apply, indicate all) ⁶

According to International Patent Classification (IPC) or to both National Classification and IPC

Int.Cl⁴    A61K33/24

**II. FIELDS SEARCHED**

| Minimum Documentation Searched ⁷ | |
|---|---|
| Classification System | Classification Symbols |
| IPC | A61K33/24 |

| Documentation Searched other than Minimum Documentation to the Extent that such Documents are Included in the Fields Searched ⁸ |
|---|
| |

**III. DOCUMENTS CONSIDERED TO BE RELEVANT** ⁹

| Category * | Citation of Document, ¹¹ with indication, where appropriate, of the relevant passages ¹² | Relevant to Claim No. ¹³ |
|---|---|---|
| X | DE, A, 2162373 (Agence Nationale de Valorisation de la Recherche) 24 August 1972 (24. 08. 72) & BE, A, 776565 & FR, A, 2117803 & GB, A, 1385489 & CA, A, 962947 | 1-4 |
| X | FR, A, 2161837 (Agence Nationale de Valorisation de la Recherche) 17 August 1973 (17. 08. 73) (Family: none) | 1-4 |
| X | JP, A, 62-230619 (Polytoronics Kabushiki Kaisha) 9 October 1987 (09. 10. 87) (Family: none) | 1-4 |
| X | JP, A, 62-230620 (Polytoronics Kabushiki Kaisha) 9 October 1987 (09. 10. 87) (Family: none) | 1-4 |

\* Special categories of cited documents: ¹⁰

"A"  document defining the general state of the art which is not considered to be of particular relevance

"E"  earlier document but published on or after the international filing date

"L"  document which may throw doubts on priority claim(s) or which is cited to establish the publication date of another citation or other special reason (as specified)

"O"  document referring to an oral disclosure, use, exhibition or other means

"P"  document published prior to the international filing date but later than the priority date claimed

"T"  later document published after the international filing date or priority date and not in conflict with the application but cited to understand the principle or theory underlying the invention

"X"  document of particular relevance; the claimed invention cannot be considered novel or cannot be considered to involve an inventive step

"Y"  document of particular relevance; the claimed invention cannot be considered to involve an inventive step when the document is combined with one or more other such documents, such combination being obvious to a person skilled in the art

"&"  document member of the same patent family

**IV. CERTIFICATION**

| Date of the Actual Completion of the International Search | Date of Mailing of this International Search Report |
|---|---|
| January 18, 1989 (18. 01. 89) | January 30, 1989 (30. 01. 89) |
| International Searching Authority | Signature of Authorized Officer |
| Japanese Patent Office | |

Form PCT ISA 210 (second sheet) (January 1985)

| | FURTHER INFORMATION CONTINUED FROM THE SECOND SHEET | |
|---|---|---|
| A | DE, A, 2655690 (Agence Nationale de Valorisation de la Recherche) 23 June 1977 (23. 06. 77) & BE, A, 848836 & JP, A, 52-94431 & FR, A, 2334366 & GB, A, 1573002 & JP, B, 61-20525 | 1-4 |
| A | FR, A, 2424028 (Agence Nationale de Valorisation de la Recherche) 23 November 1979 (23. 11. 79) (Family: none) | 1-4 |

V.☐ **OBSERVATIONS WHERE CERTAIN CLAIMS WERE FOUND UNSEARCHABLE** [1]

This international search report has not been established in respect of certain claims under Article 17(2) (a) for the following reasons:

1.☐ Claim numbers        , because they relate to subject matter not required to be searched by this Authority, namely:

2.☐ Claim numbers .........., because they relate to parts of the international application that do not comply with the prescribed requirements to such an extent that no meaningful international search can be carried out, specifically:

3.☐ Claim numbers ............, because they are dependent claims and are not drafted in accordance with the second and third sentences of PCT Rule 6.4(a).

VI.☐ **OBSERVATIONS WHERE UNITY OF INVENTION IS LACKING** [2]

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

Form PCT/ISA/210 (supplemental sheet (2)) (January 1985)

| | | |
|---|---|---|
| **FURTHER INFORMATION CONTINUED FROM THE SECOND SHEET** | | |
| A | EP, A, 185584 (Institut Pasteur; Centre National de la Recherche Scientifique) 25 June 1986 (25. 06. 86) & DE, A, 3542165 & AU, A, 8550580 & JP, A, 61-227525 & US, A, 4759929 & EP, B, 185584 & DE, G, 3564907 | 1-4 |
| A | JP, A, 61-063619 (Sasano Hiroyuki) 1 April 1986 (01. 04. 86) (Family: none) | 1-4 |
| ··A | FR, A, 2372633 | 1-4 |

V.☐ **OBSERVATIONS WHERE CERTAIN CLAIMS WERE FOUND UNSEARCHABLE** [1]

This international search report has not been established in respect of certain claims under Article 17(2) (a) for the following reasons:

1.☐ Claim numbers          , because they relate to subject matter not required to be searched by this Authority, namely·

2.☐ Claim numbers     . . . ., because they relate to parts of the international application that do not comply with the prescribed requirements to such an extent that no meaningful international search can be carried out, specifically:

3.☐ Claim numbers .... ........, because they are dependent claims and are not drafted in accordance with the second and third sentences of PCT Rule 6.4(a).

VI.☐ **OBSERVATIONS WHERE UNITY OF INVENTION IS LACKING** [2]

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

Form PCT/ISA/210 (supplemental sheet (2)) (January 1985)

**FURTHER INFORMATION CONTINUED FROM THE SECOND SHEET**

| | | |
|---|---|---|
| | (Agence Nationale de Valorisation de la Recherche) 4 August 1978 (04. 08. 78) & BE, A, 861233 & DE, A, 2753312 & DE, A, 2760338 & GB, A, 1575940 & CA, A, 1124989 & CH, A, 628005 & CH, A, 628864 & JP, A, 53-92400 & JP, A, 61-165336 & JP, B, 62-21732 | |
| A | Chemical Abstracts, Abstract No. 81(25): 163871w (Progr. Immunobiol. Stand., Volume Date 1971, 5, 285-8: RAYNAUD, M.; | 1-4 |

**V.☐ OBSERVATIONS WHERE CERTAIN CLAIMS WERE FOUND UNSEARCHABLE** [1]

This international search report has not been established in respect of certain claims under Article 17(2) (a) for the following reasons:

1.☐ Claim numbers       . because they relate to subject matter not required to be searched by this Authority, namely:

2.☐ Claim numbers ........ , because they relate to parts of the international application that do not comply with the prescribed requirements to such an extent that no meaningful international search can be carried out, specifically:

3.☐ Claim numbers ............, because they are dependent claims and are not drafted in accordance with the second and third sentences of PCT Rule 6.4(a).

**VI.☐ OBSERVATIONS WHERE UNITY OF INVENTION IS LACKING** [2]

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

Form PCT/ISA/210 (supplemental sheet (2)) (January 1985)

International Application No. PCT/JP88/01280

| FURTHER INFORMATION CONTINUED FROM THE SECOND SHEET | | |
|---|---|---|
| A | CHERMANN, J. C.; PLATE, F.; JASMIN, C.; MATHE, G.; SINOUSSI, F.; "Abstract of properties of new inhibitors of murine leukemia and sarcoma viruses") Chemical Abstracts, Abstract No.82(9): 51628m (J. Natl. Cancer Inst., 53(2), 469-74 (1974),; JASMIN, C.; CHERMANN, J.C. ; HERVE, G.; TEZE, A.; SOUCHAY, P.; BOY-LOUSTAU, C.; RAYBAUD, N; SINOUSSI, F.; RAYNAUD, M.: "Abstract of in vivo inhibition of murine leukemia and sarcoma viruses by the heteropolyanion | 1-4 |

**V.☐ OBSERVATIONS WHERE CERTAIN CLAIMS WERE FOUND UNSEARCHABLE [1]**

This international search report has not been established in respect of certain claims under Article 17(2) (a) for the following reasons:

1.☐ Claim numbers ..... , because they relate to subject matter not required to be searched by this Authority, namely:

2.☐ Claim numbers ........., because they relate to parts of the international application that do not comply with the prescribed requirements to such an extent that no meaningful international search can be carried out, specifically:

3.☐ Claim numbers ..........., because they are dependent claims and are not drafted in accordance with the second and third sentences of PCT Rule 6.4(a).

**VI.☐ OBSERVATIONS WHERE UNITY OF INVENTION IS LACKING [2]**

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

Form PCT/ISA/210 (supplemental sheet (2)) (January 1985)

**FURTHER INFORMATION CONTINUED FROM THE SECOND SHEET**

5-tungsto-2-antimonate")

---

**V.☐ OBSERVATIONS WHERE CERTAIN CLAIMS WERE FOUND UNSEARCHABLE [1]**

This international search report has not been established in respect of certain claims under Article 17(2) (a) for the following reasons:

1.☐ Claim numbers        , because they relate to subject matter not required to be searched by this Authority, namely:

2.☐ Claim numbers ....... .., because they relate to parts of the international application that do not comply with the prescribed requirements to such an extent that no meaningful international search can be carried out, specifically:

3.☐ Claim numbers ........ ..., because they are dependent claims and are not drafted in accordance with the second and third sentences of PCT Rule 6.4(a).

---

**VI.☐ OBSERVATIONS WHERE UNITY OF INVENTION IS LACKING [2]**

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

---

Form PCT/ISA/210 (supplemental sheet (2)) (January 1985)